(19) Europäisches Patentamt
European Patent Office
Office européen des brevets

(11) Veröffentlichungsnummer : **0 041 675**
**B1**

(12) # EUROPÄISCHE PATENTSCHRIFT

(45) Veröffentlichungstag der Patentschrift : **11.12.85**

(21) Anmeldenummer : **81104170.6**

(22) Anmeldetag : **01.06.81**

(51) Int. Cl.4 : **C 07 D263/34**, C 07 D413/04, C 07 D413/10, C 07 D413/14, G 03 C 1/727// G03F7/00

(54) **4-Halogen-5-(trichlormethyl-phenyl)-oxazol-Derivate, ein Verfahren zu ihrer Herstellung und sie enthaltende strahlungsempfindliche Massen.**

(30) Priorität : **09.06.80 DE 3021590**

(43) Veröffentlichungstag der Anmeldung : **16.12.81 Patentblatt 81/50**

(45) Bekanntmachung des Hinweises auf die Patenterteilung : **11.12.85 Patentblatt 85/50**

(84) Benannte Vertragsstaaten : **BE DE FR GB IT NL SE**

(56) Entgegenhaltungen :
**EP-A- 0 010 652**
**DE-A- 2 820 655**
**US-A- 3 912 606**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Dönges, Reinhard, Dr.**
**Oranienstrasse 1**
**D-6232 Bad Soden (DE)**

**EP 0 041 675 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 4-Halogen-5-(trichlormethylphenyl)-oxazol-Derivate, die in der 2-Stellung des Oxazol-Rings substituiert sind ; sie betrifft außerdem ein Verfahren zur Herstellung dieser Verbindungen und diese Verbindungen enthaltende strahlungsempfindliche Massen.

Halogenmethylgruppen aufweisende Verbindungen spielen seit längerer Zeit eine wichtige Rolle als Vor-, Zwischen- und Endprodukte auf zahlreichen Sachgebieten, beispielsweise als Pharmazeutika oder als Bestandteile von strahlungsempfindlichen Massen.

Als Pharmazeutika mit Wirksamkeit gegen Malaria sind beispielsweise Hexachlor-p-xylol

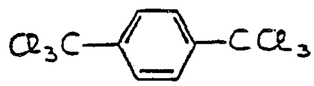

oder 2-(Trichlormethyl-phenyl)-5-trichlormethyl-1.3.4-oxadiazole

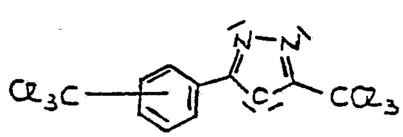

zu nennen (siehe G. Ehrhart und H. Ruschig, « Arzneimittel », Verlag Chemie — Weinheim, 1972, S. 197/198).

In Strahlungsempfindlichen Massen können die Halogenmethylgruppen tragenden Verbindungen als Photoinitiatoren eingesetzt werden, d. h. es werden einerseits die bei Einwirkung von Strahlung aus ihnen entstehenden Radikale zur Auslösung von Polymerisationsreaktionen, Vernetzungsreaktionen oder farblichen Veränderungen ausgenutzt oder es werden andererseits durch die von ihnen freigesetzte Säure Folgereaktionen bewirkt. Zu den seit längerem bekannten Photoinitiatoren zählen beispielsweise Tetrabrommethan $CBr_4$, Tribrom-acetophenon $Br_3C—CO—C_6H_5$ und Jodoform $CHJ_3$. Diese relativ leicht zugänglichen Verbindungen absorbieren jedoch nur kurzwelliges UV-Licht, wodurch sie im Anregungsbereich der in der Reproduktionstechnik gebräuchlichen Belichtungslampen nur eine geringe spektrale Empfindlichkeit aufweisen und deshalb mit Hilfe eines zusätzlichen Sensibilisators angeregt werden müssen.

Es wurde versucht, die vorher genannten Schwierigkeiten durch den Einsatz von bestimmte Chromophore aufweisenden halogenorganischen Verbindungen zu überwinden. Aus dem Stand der Technik sind beispielsweise die folgenden Druckschriften bekannt geworden :

Aus der DE-B-19 49 010 ist die Verwendung von halogenmethylierten Benzophenonen als Initiatoren der Photopolymerisation ungesättigter Verbindungen bekannt, ein Beispiel für einen solchen Photoinitiator ist die folgende Verbindung :

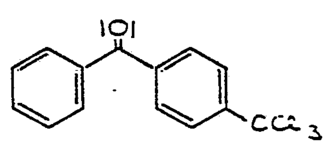

In der DE-C-20 27 467 wird eine photopolymerisierbare Kopiermasse beschrieben, die eine ethylenisch-ungesättigte polymerisierbare Verbindung, ein Bindemittel und als Photoinitiator eine gegebenenfalls substituierte Verbindung der Acridin- oder Phenazinreihe enthält.

Die s-Triazinderivate der DE-A-22 43 621 weisen mindestens eine Trihalogenmethylgruppe und mindestens eine chromophore Gruppierung auf, die mit dem Triazinring über ethylenisch-ungesättigte Gruppen ein konjugiertes System bildet ; sie sind als Photoinitiatoren in Massen wirksam, die eine ethylenisch-ungesättigte, zur durch Radikale initiierten Additionspolymerisation befähigte Verbindung enthalten. Ein Beispiel für einen solchen Photoinitiator ist die folgende Verbindung :

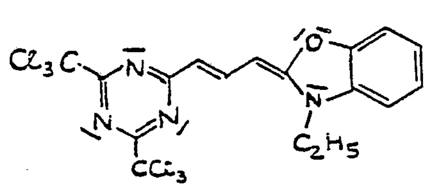

Das durch Belichten löslich werdende Stoffgemisch gemäß der DE-B-23 06 248 enthält eine

wasserunlösliche, durch Einwirkung einer photolytisch gebildeten Säure löslich werdende Verbindung (Aryl-alkyl-acetale und Aryl-alkyl-aminale) und als Photoinitiator eine unter Normalbedingungen neutral reagierende stabile halogenhaltige organische Verbindung, die photolytisch spaltbar ist und dabei eine Säure liefert. Beispiele für geeignete Photoinitiatoren sind : Tetrabromkohlenstoff, Hexabromethan, Trichloracetophenon, Halogenmethyl-s-triazine oder Vinylhalogenmethyl-s-triazine (letztere siehe auch DE-A-22 43 621).

Aus der DE-A-27 18 259 ist eine strahlungsempfindliche Masse bekannt, die als strahlungsempfindliche Verbindung ein s-Triazinderivat mit mindestens einer Halogenmethylgruppe und einem zwei- oder mehrkernigen aromatischen Rest als Substituenten enthält ; ein Beispiel für eine solche strahlungsempfindliche Verbindung ist die folgende Verbindung :

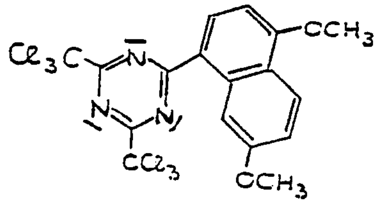

In der DE-A-28 51 472 wird eine lichtempfindliche Masse beschrieben, die als Photoinitiator ein 2-Halogenmethyl-5-vinyl-1,3,4-oxadiazol-Derivat enthält ; ein Beispiel für einen solchen Photoinitiator ist die folgende Verbindung :

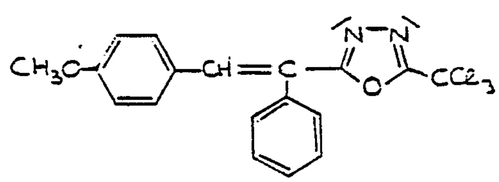

Die Photoinitiatoren der UV-empfindlichen Massen gemäß der US-A-3 912 606 sind Halogenmethylgruppen tragende Benzoxazole, die diese Halogenmethylgruppe direkt oder über einen Benzolring gebunden in der 2-Stellung aufweisen ; ein Beispiel für einen solchen Photoinitiator ist die folgende Verbindung :

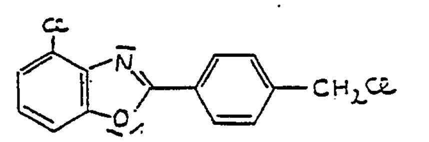

Die aus dem Stand der Technik bekannten Photoinitiatoren bzw. strahlungsempfindlichen Verbindungen weisen aber einen oder mehrere der folgenden Nachteile auf :

— die kein photolytisch-spaltbares Halogen aufweisenden Verbindungen können für säurekatalysierte Molekülveränderungen nicht eingesetzt werden (z. B. DE-A-20 27 467),

— bestimmte Synthesen führen in befriedigender Weise nur zu Monohalogenmethyl-substituenen im Molekül (z. B. US-A-3 912 606),

— die Absorptionsmaxima sind zwar gegenüber den einfachen bekannten Photoinitiatoren verschoben, die Absorption ist jedoch immer noch relativ zu kurzwellig (z. B. US-A-3 912 606);

— die Reaktionsbedingungen zur Erzeugung der Verbindungen sind verhältnismäßig drastisch, so daß die Reaktionsausbeute gering ist und die Bildung von unerwünschten Nebenprodukten erleichtert wird (z. B. DE-A-22 43 621, DE-A-27 18 259 oder DE-A-28 51 472), oder

— der Einsatz von bestimmten Katalysatoren erlaubt nur die Anwesenheit von wenigen bestimmten funktionellen Gruppen im Molekül (z. B. DE-A-27 18 259).

Aus der EP-A-10 652 sind 4-Chlor-1,3-oxazole bekannt, die in 2- und 5-Stellung gegebenenfalls substituierte aromatische Reste tragen. Als Substituenten werden keine Trichlormethylgruppen offenbart. Die Verbindungen sind als optische Aufheller und Photoleiter geeignet.

Aus der DE-A-28 20 655 sind Isoxazole bekannt, die durch Trifluormethylgruppen enthaltende aromatische Reste substituiert sind. Die Verbindungen sind als Mittel zur Regulierung des Pflanzenwachstums geeignet.

Aufgabe der vorliegenden Erfindung ist es deshalb, neue Verbindungen zu synthetisieren, die insbesondere strahlungsempfindlich sind und damit bevorzugt auf dem sich z. Zt. rasch weiterentwickelnden Gebiet der Reproduktionstechnik eingesetzt werden können. Die Verbindungen sollen verhältnismäßig einfach zugänglich sein und eine große Breite an Variationsmöglichkeiten bieten, um für

die verschiedensten Bedürfnisse auf dem jeweiligen Anwendungsgebiet optimal angepaßt werden zu können ; sie sollen beispielsweise eine spektrale Empfindlichkeit aufweisen, die relativ zur Emission herkömmlicher Strahlungsquellen einen breiten Absorptionsbereich umfaßt, d. h. insbesondere im ultravioletten und kurzwelligen sichtbaren Bereich des Lichtes empfindlich sein. Zusätzlich sollen die Verbindungen, sofern sie in strahlungsempfindlichen Massen auf dem Reproduktionssektor (beispielsweise auf Druckplatten) eingesetzt werden, bereits nach der Bestrahlung eine deutlich sichtbare farbige Abbildung der Vorlage erzeugen können, wodurch beispielsweise noch vor der eigentlichen Entwicklung der strahlungsempfindlichen Masse Korrekturen von Belichtungsfehlern ermöglicht werden.

Die erfindungsgemäße Lösung dieser Aufgabe sind :

4-Halogen-5-(trichlormethyl-phenyl)-oxazol-Derivate der allgemeinen Formel (I)

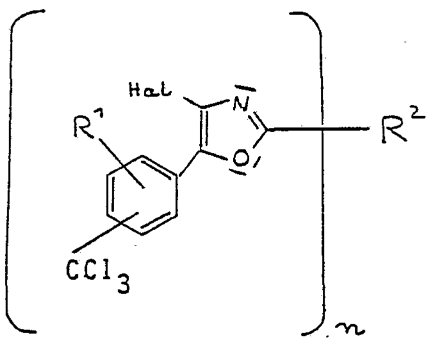

(I)

in der bedeuten

Hal ein Halogenatom

n die Zahl 1 oder 2

$R^1$ ein Wasserstoffatom oder eine weitere Gruppe $CCl_3$ und

$R^2$ einen n-wertigen, unsubstituierten oder durch Dialkylaminogruppen mit 2 bis 6 Kohlenstoffatomen, Halogenatome, Nitro-, Cyano-, Sulfonyl-, Carboxy-, Trifluormethyl-, Alkyl-, Cycloalkyl-, Alkoxy-, Alkoxycarbonyl-, Alkoxycarbonimidoyl (Carbalkoxyimino-), Carboxyamido-, Phenyl-, Naphthyl-, Indenyl-, Fluorenyl-, Anthryl-, Phenanthryl-, Pyrenyl-, Biphenylyl-, Stilbenyl-, Styryl-, Furyl-, Benzofuryl-, Dibenzofuryl-, Pyrrolyl-, Indolyl-, Carbazolyl-, Thienyl-, Benzothienyl-, Imidazolyl-, Benzimidazolyl-, Oxazolyl-, Benzoxazolyl-, Isoxazolyl-, Thiazolyl-, Benzthiazolyl-, Triazolyl-, Oxdiazolyl-, Thiadiazolyl-, Pyridyl-, Chinolyl-, Isochinolyl-, Acridyl-, Pyrimidyl-, Benzopyranyl-, Benzothianyl- oder Phenoxygruppen substituierten, maximal vierkernigen aromatischen oder heteroaromatischen, gegebenenfalls partiell hydrierten Rest, der an einem ungesättigten Ring-C-Atom direkt oder über eine bis zu vier ausschließlich olefinisch ungesättigten C-Atome enthaltenden Kette mit dem Oxazolyl-Teil des Moleküls gemäß der allgemeinen Formel (I) verbunden ist.

In einer bevorzugten Ausführungsform der Erfindung bedeuten in der allgemeinen Formel (I) Hal ein Chlor- oder Bromatom und n die Zahl 1 oder 2. Besonders bevorzugt werden Verbindungen der allgemeinen Formel I, in denen Hal ein Chloratom, n die Zahl 1, $R^1$ ein Wasserstoffatom und $R^2$ einen durch die oben genannten Substituenten substituierten oder unsubstituierten Phenyl-, Benzofuranyl- oder Naphthylrest bedeutet.

Beispiele für den Rest $R^2$ sind die Reste : Phenyl, Naphthyl, Indenyl, Fluorenyl, Anthryl, Phenanthryl, Pyrenyl, Biphenylyl, Stilbenyl, Styryl, Furyl, Benzofuryl, Dibenzofuryl, Pyrrolyl, Indolyl, Carbazolyl, Thienyl, Benzothienyl, Imidazolyl, Benzimidazolyl, Oxazolyl, Benzoxazolyl, Isoxazolyl, Thiazolyl, Benzthiazolyl, Triazolyl, Oxdiazolyl, Thiadiazolyl, Pyridyl, Chinolyl, Isochinolyl, Acridyl, Pyrimidyl, Benzopyranyl und Benzo-thianyl.

Der Rest $R^2$ kann bevorzugt bis zu vier Substituenten tragen.

Bevorzugte Substituenten am Phenylrest in 5-Stellung des Oxazol-Derivates sind ein Trichlormethylrest in 4-Stellung des Phenylrings oder zwei Trichlormethylreste in 3- und 5-Stellung des Phenylrings.

Es ist bekannt, daß sich 4-Halogen-2,5-diaryloxazole durch Einleiten von Halogenwasserstoff bei 0° C in eine Lösung eines Aroylcyanids und eines aromatischen Aldehyds in Ether und anschließende Hydrolyse darstellen lassen [siehe M. Davis, R. Lakhan und B. Ternai, J. Heterocycl. Chem. *14*, 317, (1977)] ; nach der Lehre der DE-OS 28 44 394 ist diese Methode nicht auf aromatische Aldehyde als Ausgangsmaterial beschränkt.

Die erfindungsgemäßen trichlormethyl-phenylsubstituierten Oxazolderivate lassen sich in vorteilhafter Weise aus einem trichlormethylsubstituierten Benzoylcyanid der allgemeinen Formel II und einem Aldehyd der allgemeinen Formel III herstellen, wobei die folgende Reaktionsgleichung den Reaktionsverlauf symbolisiert (die Bezeichnungen Hal, n, $R^1$ und $R^2$ haben die weiter oben angegebene Bedeutung) :

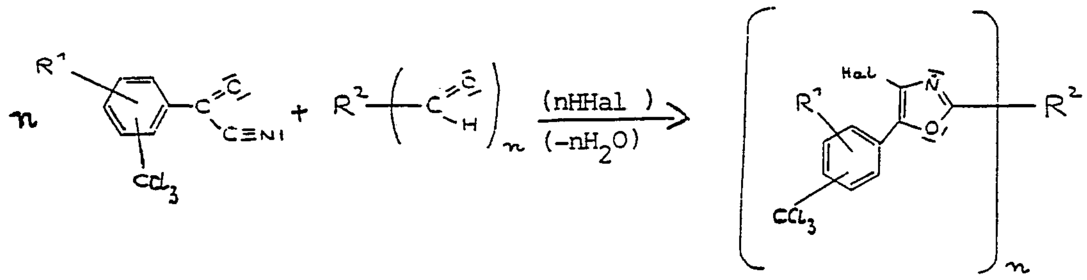


(II) (III) (I)

Die bei dieser unter milden Bedingungen in einem inerten, bevorzugt einen Halogenwasserstoff gut solvatisierenden organischen Lösemittel wie z. B. Tetrahydrofuran, Diethylether, Diisopropylether, Diethylenglykol-diethyl-ether bei Temperaturen zwischen − 30 °C und + 20 °C, bevorzugt − 20 °C bis 0 °C, ablaufenden Reaktion einzusetzenden trichlormethylsubstituierten Benzoylcyanide lassen sich aus den entsprechenden Säurechloriden mit geeigneten Metallcyaniden wie NaCN, KCN oder CuCN herstellen. Die meisten hierzu bekannten Verfahren besitzen jedoch den Nachteil, daß sie hohe Reaktionstemperaturen verlangen [siehe beispielsweise Organic Synthesis, Coll. Vol. III 112-114, (1955), DE-A-26 42 140, 27 08 182 und 27 08 183, Angew. Chem. *68*, 425 (1956)], wodurch es leicht zur Zerstörung der Trichlormethylgruppen kommen kann.

Es wurden in der Literatur auch Umsetzungen unter den Bedingungen der « Phasen-Transfer-Katalyse » bei niedrigeren Temperaturen beschrieben [siehe z. B. Tetrahedron Lett. *26*, 2275 (1974)], wobei jedoch über eine starke Bildung der « dimeren » Benzoylcyanide berichtet wird, falls die eingesetzten Verbindungen elektronenanziehende Substituenten tragen, und damit den nucleophilen Angriff von Cyanidionen an der Carboxylgruppe erleichtern.

Es empfiehlt sich daher, die zur Darstellung der erfindungsgemäßen Verbindungen als Vorstufen dienenden trichlormethylsubstituierten Benzoylcyanide zwar mit Hilfe eines Alkalimetallcyanids unter « Phasen-Transfer-Bedingungen », jedoch unter gleichzeitiger Abpufferung des Cyanids mit Blausäure HCN durchzuführen [siehe dazu DE-A-27 17 075].

Zur Darstellung der erfindungsgemäßen Verbindungen können nahezu alle Aldehyde als zweite Komponente neben den Benzoylcyaniden eingesetzt werden, sofern sie keine säureempfindliche Gruppierung tragen. Besonders geeignet sind Aldehyde, die den Chromophor des 5-Phenyloxazolsystems verlängern, so daß die Verbindungen eine sich mit der Emission der gebräuchlichen Belichtungslampen überlagernde Absorption im Bereich von 250 bis 500 nm, bevorzugt 350 bis 400 nm, erhalten. Vorzugsweise werden daher aromatische, olefinische und heterocyclisch-ungesättigte Aldehyde eingesetzt, beispielsweise sind folgende Verbindungen geeignet :

4-Acetylbenzaldehyd, 5-Acetyl-2,4-dimethoxybenzaldehyd,
4-Ethansulfonylbenzaldehyd, 2-, 3- und 4-Ethoxybenzaldehyd,
4-Ethoxy-3-methoxybenzaldehyd,
3- und 4-Ethoxycarbonylbenzaldehyd,
4-Acetamidobenzaldehyd, 2-, 3- und 4-Anisaldehyd, Benzaldehyd,
5-Brom-o-anisaldehyd,
2-, 3- und 4-Brombenzaldehyd,
2-Brom-4-cyanobenzaldehyd,
4-sek. Butylbenzaldehyd, p-(Benzoxazolyl-2)-benzaldehyd, 2-, 3- und 4-Chlorbenzaldehyd,
2-Chlor-4-dimethylaminobenzaldehyd,
2-Chlor-6- fluorbenzaldehyd, 2-, 3- und 4-Cyanobenzaldehyd,
4′-Cyano-stilben-4-aldehyd, 4-Diethylaminobenzaldehyd,
3,5-Di-Tert.butyl-benzaldehyd,
2,3-, 2,4- und 2,6-Dichlorbenzaldehyd,
3,4- und 3,5-Dichlorbenzaldehyd,
4,4′-Diformyldiphenylether, 2,4-Diethoxy-benzaldehyd,
3,5-Dimethyl-4-nitrobenzaldehyd,
2,4- und 2,5-Dimethoxybenzaldehyd,
3,4- und 3,5-Dimethoxybenzaldehyd,
4-Dimethylaminobenzaldehyd,
2,3- und 2,5-Dimethyl-p-anisaldehyd,
2,4-, 2,5- und 2,6-Dimethylbenzaldehyd,
3,5-Dimethylbenzaldehyd, 3-Fluor-p-anisaldehyd,
2-, 3- und 4-Fluorbenzaldehyd,
p-(Imidazolyl-1)-benzaldehyd, 4-Isopropylbenzaldehyd,
4-Jodbenzaldehyd, Mesitaldehyd,

3-Methyl-p-anisaldehyd,
2-Methoxy-5-acetylbenzaldehyd, 4-Methylsulfonbenzaldehyd,
3-Methoxycarbonylbenzaldehyd, 4'-Methoxystilben-4-aldehyd,
3- und 4-Nitrobenzaldehyd, 5-Nitroveratraldehyd, Pentafluorbenzaldehyd, 2-, 3- und 4-Phenoxybenzaldehyd, Piperonal, Stilben-4-aldehyd,
3- und 4-Trifluormethylbenzaldehyd,
2,4,6-Tricyano-3,5-dimethylbenzaldehyd,
2,4,6-Triethylbenzaldehyd, 2,3,5,6-Tetramethylbenzaldehyd,
2,3,5-Trichlorbenzaldehyd, 2,4,5-Trichlorbenzaldehyd,
2,4,6-Trichlorbenzaldehyd,
2,3,4-Trimethoxybenzaldehyd, 2,4,5-Trimethoxybenzaldehyd,
2,4,6-Trimethoxybenzaldehyd, 3,4,5-Trimethoxybenzaldehyd,
2-, 3- und 4-Tolylaldehyd, Terephthalaldehyd, iso-Phthalaldehyd,
2-Methoxy-5-methylisophthalaldehyd, 5-Nitroisophthalaldehyd, Tetramethylisophthalaldehyd, 2,5-Dichlorterephthalaldehyd,
2,5-Diethoxyterephthalaldehyd, 2,5-Dimethylterephthalaldehyd, Nitroterephthalaldehyd, 2- und 4-Biphenylaldehyd,
2,2'-, 3,3'- und 4,4'-Diphenyldicarboxaldehyd ;
5,5'-Diethyl-4,4', 6,6'-tetramethoxybiphenyl-3,3'-dialdehyd,
p-Terphenyl-4-aldehyd, Dibenzofulven-9-aldehyd, Zimtaldehyd, α-Chlor- und α-Brom-zimtaldehyd,
4-Dimethylaminozimtaldehyd, 2- und 4-Nitrozimtaldehyd,
α-Methylzimtaldehyd, β-Phenylzimtaldehyd
β-(1-Naphthyl)acrolein, β-(4-Methoxy-1-naphthyl)acrolein,
4-Chlor-$\Delta^3$-chromen-3-aldehyd,
4-Chlor-1,2-dihydro-naphthalin-3-aldehyd,
4,6-Dichlor-$\Delta^3$-thiochromen-3-aldehyd,
5,6,7-Trimethoxy-1,2-dihydronaphthalin-3-aldehyd,
1-, 2-, und 9-Anthraldehyd, 10-Methyl-9-anthraldehyd,
10-Methoxy-9-anthraldehyd, 10-Brom-9-anthraldehyd,
2- und 10-Chlor-9-anthraldehyd, 1,8-Anthracendialdehyd,
7-, 10- und 12-Benz[a]anthracenaldehyd,
1-, 2- und 4-Fluorenaldehyd,
2,7-Fluorendialdehyd, 3-Methylinden-2-aldehyd,
Inden-2-aldehyd, 1,1-Dimethylinden-2-aldehyd,
6-Methoxyinden-2-aldehyd, 1- und 2-Naphthaldehyd,
5-Nitro-1-naphthaldehyd,
1,3-, 1,4-, 1,5-, 1,6-, 1,7-, 2,6- und 2,7-Naphthalindialdehyd,
2-, 4- und 5-Methoxy-1-naphthaldehyd,
6-Methoxy-2-naphthaldehyd, 4-Chlor-1-naphthaldehyd, Acenaphthen-5-aldehyd, Acenaphthylen-1-aldehyd,
1-, 2-, 3- und 9-Phenanthrenaldehyd,
3-Methoxyphenanthren-9-aldehyd, 1- und 3-Pyrenaldehyd,
2-, 3- und 4-Pyridinaldehyd,
2-, 4-, 5- und 6-Methylpyridin-3-aldehyd,
2,6-Dimethylpyridin-3-aldehyd,
4-Methylpyridin-2,6-dialdehyd,
2,6-Dichlorpyridin-4-aldehyd, 6-Methylpyridin-2-aldehyd,
2-, 3-, 4-, 6-, 7- und 8-Chinolinaldehyd,
6,7-Dimethoxy-4-phenylchinolin-2-aldehyd,
6-Ethoxichinolin-2-aldehyd, 6,7-Dimethylchinolin-2-aldehyd,
1-, 3-, 4-, 5- und 7-Isochinolinaldehyd,
6,7-Dimethoxyisochinolin-1-aldehyd,
9-Ethylcarbazol-3-aldehyd, Acridin-9-aldehyd,
2-, 3- und 5-Benzofuranaldehyd,
5-Methyl-3-phenylbenzofuran-2-aldehyd,
3-(β-Naphthyl)benzofuran-2-aldehyd,
2,3-Dimethylbenzofuran-4-aldehyd, 2- und 3-Furaldehyd,
5-Chlorfurfural, 1- und 2-Dibenzofuranaldehyd, Dibenzofuran-2,8-dialdehyd, 5-Methylfurfural,
6-Methoxybenzofuran-2-aldehyd,
4,6-Dimethoxybenzofuran-2-aldehyd,
2-Phenylbenzofuran-3-aldehyd, 2,5-Furandialdehyd,
5-Nitrofuran-2-aldehyd, 1,3,5-Trimethylpyrrol-2-aldehyd,
1,3,5-Trimethylpyrrol-2,4-dialdehyd,
1-Methylpyrrol-2-aldehyd, 1-Methylpyrrol-2,5-dialdehyd,
1-Methylimidazol-2- und -4-aldehyd,

1-Methylindol-2-aldehyd, 1-Methyl-2-phenylindol-3-aldehyd,
4-Oxazolaldehyd, 2-Benzoxazolaldehyd, 2-Naphthoxazolaldehyd, Benzbisoxazol-2,6-dialdehyd, 3 Methylisoxazol-5-aldehyd,
3-Phenylisoxazol-5-aldehyd, 2- und 3-Thiophenaldehyd,
5-Bromthiophen-2-aldehyd, 5-Methylthiophen-2-aldehyd,
2- und 3-Dibenzothiophenaldehyd, Dibenzothiophen-2,8-dialdehyd,
2-, 3-, 4-, 5-, 6- und 7-Benzothiophenaldehyd,
3-Brombenzothiophen-7-aldehyd,
2- und 4-Thiazolaldehyd, 4-Methylthiazol-2-aldehyd, Benzthiazol-2-aldehyd, 6-Nitrobenzthiazol-aldehyd, Naphthiazol-2-aldehyd, Benzbisthiazol-2,6-dialdehyd,
5-Phenyl-1,3,4-oxdiazol-2-aldehyd, 1,3,4-Thiadiazol-2-aldehyd,
5-Phenyl-1,3,4-thiadiazol-2-aldehyd,
5-Methyl-1,3,4-thiadiazol-2-aldehyd, Cumarin-3-aldehyd, Chinazolin-2-aldehyd,
2,4,6-Trimethoxypyrimidin-4-aldehyd,
2,4,6-Trichlorpyrimidin-4-aldehyd.

Außerdem sind chromophorsubstituierte Aldehyde geeignet, wie sie zur Herstellung von Cyaninfarbstoffen verwendet werden (siehe beispielsweise The Chemistry of Heterocycl. Comp. Band 18, S. 115 ff., Interscience Publishers, 1964) oder in optischen Aufhellern zum Einsatz kommen.

Die Molverhältnisse der Reaktanden können in weiten Grenzen variiert werden, vorzugsweise wird im Bereich zwischen 0,8 und 1,2 Mol für die eine Komponente pro Mol der anderen Komponente gearbeitet, besonders bevorzugt ist dabei ein ca. 10 %iger Überschuß an Benzoylcyanid gegenüber dem eingesetzten Aldehyd.

Zu den aus den oben näher beschriebenen Komponenten hergestellen erfindungsgemäßen Verbindungen zählen insbesondere die folgenden, die eine gute Strahlungsempfindlichkeit aufweisen :
4-Chlor-5-(p-trichlormethyl-phenyl)-oxazole, die folgende Substituenten aufweisen :

2-(p-Methoxyphenyl), 2-(3,4,5-Trimethoxyphenyl),
2-(2,4,6-Trimethoxyphenyl),
2-(p-Methoxycarbonylphenyl), 2-(p-Nitrophenyl),
2-(2,4-Dichlorphenyl), 2(-p-Biphenylyl), 2-(2-Fluorenyl),
2-(p-Benzoxazolyl-phenyl), 2-Stilbenyl, 2-(1-Naphthyl),
2-(2-Naphthyl), 2-(5-Acenaphthenyl),
2-(4-Methoxy-1-naphthyl), 2-(6-Methoxy-2-naphthyl),
2-(9-Phenanthrenyl), 2-(2-Benzofuranyl),
2-(5-Methoxy-2-benzofuranyl), 2-Styryl,
2-(1-Chlor-3,4-dihydro-2-naphthyl)

und die Verbindungen

1,3-Bis-[4-chlor-5-(p-trichlormethylphenyl)-2-oxazolyl]-benzol,
2,5-Bis-[4-chlor-5-(p-trichlormethylphenyl)-2-oxazolyl]-furan,
4,4'-Bis-[4-chlor-5-(p-trichlormethylphenyl)-2-oxazolyl]-diphenylether.

Die neuen Verbindungen besitzen wegen ihrer Strahlungsempfindlichkeit eine breites Anwendungsspektrum. So sind sie beispielsweise als hochwirksame Starter für Photopolymerisationsreaktionen verwendbar, die durch freie Radikale ausgelöst werden. Geeignete Monomere, die entsprechende Polyadditionen eingehen, sind beispielsweise Mono-, Bis-, Tris- und Tetra-acrylate und -methacrylate von mono- bzw. polyfunktionellen Alkoholen oder Phenolen, Acryl- und Methacrylsäure-amide, abgeleitet von mono- bzw. polyfunktionellen Aminen, Vinylester und Vinylamide. Derartige polymerisierbare Massen können noch zusätzlich Füllstoffe, Bindemittel, Polymerisationsinhibitoren, Farbstoffe, Farbvorläufer, Weichmacher, Haftvermittler oder Sauerstoffänger in unterschiedlichen Mengen beinhalten. Sind diese Massen in Schichtform auf gegebenenfalls chemisch vorbehandelte Träger aufgebracht, d. h. beispielsweise auf Metallfolien aus Stahl, Aluminium, Chrom, Kupfer, Messing, Kunststoff oder Papier, auf Glas, Holz oder Keramik oder auf Verbundmaterialien zweier oder mehrerer dieser Stoffe, kann die lichtempfindliche Schicht auch noch mit einer Deckschicht, die den Zutritt von Sauerstoff hemmt, versehen sein.

Die strahlungsempfindlichen Verbindungen sind als Photoinitiatoren bereits in Konzentrationen von etwa 0,1 % des Gesamtfeststoffs der Masse wirksam, eine Erhöhung über 10 % ist im allgemeinen unzweckmäßig. Vorzugsweise werden Konzentrationen von 1 bis 5 % verwendet.

Weiterhin können die erfindungsgemäßen Verbindungen auch in solchen strahlungsempfindlichen Massen eingesetzt werden, deren Eigenschaftsänderung durch saure Katalysatoren, die bei der Photolyse des Initiators entstehen, eingeleitet wird. Zu nennen sind etwa die kationische Polymerisation von Systemen, die Vinylether, N-Vinylverbindungen wie N-Vinylcarbazol oder spezielle säurelabile Lactone enthalten, wobei nicht ausgeschlossen wird, daß bei einigen dieser Systeme auch radikalische Vorgänge beteiligt sind. Als durch Säuren härtbare Massen sind weiterhin Aminoplaste wie Harnstoff/Formaldehydharze, Melamin/Formaldehydharze und andere N-Methylolverbindungen sowie Phenol/Formaldehydharze zu nennen. Wenn auch die Härtung von Epoxyharzen im allgemeinen durch Lewis-Säuren bzw. solche Säure erfolgt, deren Anionen eine geringere Nukleophilie als Chlorid und Bromid besitzen, also als die Anionen der bei der Photolyse der neuen Verbindungen entstehenden Halogenwasserstoffsäuren, so härten doch Schichten, die aus Epoxyharzen und Novolaken bestehen, in Gegenwart der erfindungsgemäßen Verbindungen glatt aus.

Eine weitere vorteilhafte Eigenschaft der neuen Verbindungen besteht in ihrer Fähigkeit, in gefärbten Systemen bei der Photolyse Farbumschläge hervorzurufen; aus Farbvorläufern, z. B. Leukoverbindungen, Farbbildung zu induzieren oder bathochrome Farbverschiebungen und -vertiefungen in Gemischen zu bewirken, die Cyanin-, Merocyanin- oder Styrylfarbbasen enthalten. Auch kann z. B. in den in der DE-A-15 72 080 beschriebenen Gemischen, die Farbbase, N-Vinylcarbazol und einen Halogenkohlenwasserstoff enthalten, die Halogenverbindung Tetrabrommethan durch einen Bruchteil ihrer Menge an erfindungsgemäßer Verbindung ersetzt werden. Farbumschläge sind in der Technik auch z. B. bei der Herstellung von Druckformen erwünscht, um nach der Belichtung bereits vor der Entwicklung das Kopierergebnis beurteilen zu können.

Statt der in den DE-A-23 31 377 und DE-A-26 41 100 genannten Säurespender sind vorteilhaft die vorliegenden Verbindungen zu benutzen.

Ein besonders bevorzugtes Anwendungsgebiet für die erfindungsgemäßen Verbindungen sind Massen, die neben ihnen als wesentliche Komponente eine Verbindung mit mindestens einer durch Säure spaltbaren C—O—C-Gruppierung enthalten. Als durch Säure spaltbare Verbindungen sind in erster Linie zu nennen:

A) solche mit mindestens einer Orthocarbonsäureester- und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können und

B) Polymerverbindungen mit wiederkehrenden Acetal- und/oder Ketalgruppierungen, bei denen beide α-C-Atome der zum Aufbau dieser Gruppierungen erforderlichen Alkohole aliphatisch sind.

Durch Säure spaltbare Verbindungen des Typs A als Komponenten strahlungsempfindlicher Kopiermassen sind in den DE-A-26 10 842 oder DE-A-29 28 636 ausführlich beschrieben; Kopiermassen, die Verbindungen des Typs B enthalten, sind Gegenstand der DE-B-27 18 254.

Als weitere durch Säure spaltbare Verbindungen sind z. B. noch die speziellen Aryl-alkyl-acetale und -animale der DE-B-23 06 248 zu nennen, die durch die Photolyseprodukte der erfindungsgemäßen Verbindungen ebenfalls abgebaut werden.

Solche Massen, in denen durch Einwirkung von aktinischer Strahlung mittelbar oder unmittelbar Moleküle, deren Anwesenheit die chemischen und/oder physikalischen Eigenschaften der Masse wesentlich beeinflußt, in kleinere umgewandelt werden, weisen an den bestrahlten Stellen im allgemeinen eine erhöhte Löslichkeit, Klebrigkeit oder Flüchtigkeit auf. Diese Partien können durch geeignete Maßnahmen, beispielsweise Herauslösen mit einer Entwicklungsflüssigkeit, entfernt werden. Bei kopiermassen spricht man in diesen Fällen von positiv arbeitenden Systemen.

Die bei vielen Positiv-Kopiermassen bewährten Novolak-Kondensationsharze haben sich als Zusatz auch bei der Anwendung der erfindungsgemäßen Verbindungen in Kopiermassen mit durch Säure spaltbaren Verbindungen als besonders brauchbar und vorteilhaft erwiesen. Sie fördern die starke Differenzierung zwischen den belichteten und unbelichteten Schichtpartien beim Entwickeln, besonders die höher kondensierten Harze mit substituierten Phenolen als Formaldehyd-Kondensationspartner. Die Art und Menge der Novolak-Harze kann je nach Anwendungszweck verschieden sein; bevorzugt sind Novolak-Anteile am Gesamtfeststoff zwischen 30 und 90, besonders bevorzugt 55-85 Gew.-% Zusätzlich können noch zahlreiche andere Harze mitverwendet werden, bevorzugt Vinylpolymerisate wie Polyvinylacetate, Polyacrylate, Polyvinylether und Polyvinylpyrrolidone, die selbst durch Comonomere modifiziert sein können. Der günstigste Anteil an diesen Harzen richtet sich nach den anwendungstechnischen Erfordernissen und dem Einfluß auf die Entwicklungsbedingungen und beträgt im allgemeinen nicht mehr als 20 % vom Novolak. In geringen Mengen kann die lichtempfindliche Masse für spezielle Erfordernisse wie Flexibilität, Haftung und Glanz etc. außerdem noch Substanzen wie Polyglykole, Cellulose-Derivate wie Ethylcellulose, Netzmittel, Farbstoffe und feinteilige Pigmente sowie bei Bedarf UV-Absorber enthalten. Entwickelt wird vorzugsweise mit in der Technik üblichen wäßrig-alkalischen Entwicklern, die auch kleine Anteile organischer Lösemittel enthalten können.

Die bereits im Zusammenhang mit den photopolymerisierbaren Massen aufgeführten träger kommen ebenfalls für positiv arbeitende Kopiermassen in Frage, zusätzlich die in der Mikroelektronik üblichen Silizium- und Siliziumdioxidoberflächen.

Die Menge der als Photoinitiator eingesetzten erfindungsgemäßen Verbindungen kann in den positiv arbeitenden Kopiermassen je nach Substanz und Schicht sehr verschieden sein. Günstigere Ergebnisse werden erhalten zwischen etwa 0,1 und 10 %, bezogen auf den Gesamtfeststoff, bevorzugt sind etwa 1 bis 5 %. Für Sichten mit Dicken über 10 μm empfiehlt es sich, relativ wenig Säurespender zu verwenden.

Grundsätzlich ist elektromagnetische Strahlung mit Wellenlängen bis etwa 600 nm geeignet, in den

die erfindungsgemäße Verbindung enthaltenden strahlungsempfindlichen Massem Reaktionen der beschriebenen Art auszulösen. Der bevorzugte Wellenlängenbereich erstreckt sich von 250 bis 500 nm.

Die Vielfalt der erfindungsgemäßen Verbindungen, deren Absorptionsmaxima teilweise noch weit im sichtbaren Teil des Spektrums zu finden sind und deren Absorptionsbereich über 500 nm hinausreichen kann, gestattet es, den Photoinitiator optimal auf die verwendete Lichtquelle abzustimmen. Prinzipiell ist jedoch auch eine Sensibilisierung nicht ausgeschlossen. Als Lichtquellen sind beispielsweise zu nennen :

Röhrenlampen, Xenonimpulslampen, metallhalogendotierte Quecksilberdampf-Hochdrucklampen und Kohlebogenlampen.

Darüber hinaus ist bei den erfindungsgemäßen lichtempfindlichen Kopiermassen das Belichten in üblichen Projektions- und Vergrößerungs-Geräten unter dem Licht der Metallfadenlampen und Kontakt-Belichtung mit gewöhnlichen Glühbirnen möglich. Die Belichtung kann auch mit kohärentem Licht eines Lasers erfolgen. Geeignet für die Zwecke vorliegender Erfindung sind leistungsgerechte kurzwellige Laser, beispielsweise Argon-Laser, Krypton-Ionen-Laser, Farbstoff-Laser und Helium-Cadmium-Laser, die insbesondere zwischen 250 und 500 nm emittieren. Der Laserstrahl wird mittels einer vorgegebenen programmierten Strich- und/oder Raster-Bewegung gesteuert.

Das Bestrahlen mit Elektronenstrahlen ist eine weitere Differenzierungsmöglichkeit. Elektronenstrahlen können Kopiermassen, die eine der erfindungsgemäßen Verbindungen und eine durch Säure spaltbare verbindung enthalten, wie auch viele andere organische Materialien durchgreifend zersetzen und vernetzen, so daß ein negatives Bild entsteht, wenn die unbestrahlten Teile durch Lösemittel oder Belichten ohne Vorlage und Entwickeln entfernt werden. Bei geringerer Intensität und/oder höherer Schreibgeschwindigkeit des Elektronenstrahls bewirkt dagegen der Elektronenstrahl eine Differenzierung in Richtung höherer Löslichkeit, d. h. die bestrahlten Schichtpartien können vom Entwickler entfernt werden. Die günstigsten Bedingungen können durch Vorversuche leicht ermittelt werden.

Bevorzugte Anwendung finden die eine der erfindungsgemäßen Verbindungen enthaltenden strahlungsempfindlichen Massen bei der Herstellung von Druckformen, d. h. insbesondere Offset-, autotypischen Tiefdruck- und Siebdruckformen, in Kopierlacken und in sogenannten Trockenresists.

Die folgenden Beispiele dienen zur näheren Erläuterung der Erfindung. Dabei wird zunächst die Herstellung von zwei als Ausgangsstoff eingesetzten Trichlormethylbenzoylcyaniden beschrieben.

Danach erfolgt die Beschreibung von verschiedenen erfindungsgemäßen Verbindungen, woran sich die Anwendung von einigen dieser Verbindungen in strahlungsempfindlichen Massen anschließt.

In den Beispielen stehen Gew.-Teile (Gt) und Vol.-Teile (Vt) im Verhältnis von g zu ml. Prozent- und Mengenangaben sind, wenn nichts anderes angegeben ist, in Gewichtseinheiten zu verstehen.

## Beispiel 1

Herstellung des p-Trichlormethylbenzoylcyanids

Zu einer Lösung von 275 g p-Trichlormethylbenzoylchlorid und 2 g Dimethylbenzylamin in 1,5 l Methylenchlorid werden bei – 10 °C bis – 20 °C 50 ml Cyanwasserstoff gelöst in 150 ml Methylenchlorid getropft. Danach wird bei der gleichen Temperatur eine Lösung von 60 g Kaliumcyanid in 120 ml Wasser langsam zugetropft und schließlich das Gemisch zwei Stunden nachgerührt. Die ausgefallenen Feststoffe werden abgesaugt, die Phasen getrennt, die organische Phase mehrmals mit Wasser gewaschen über $Na_2SO_4$ getrocknet und eingeengt. Der verbleibende, ölige Rückstand wird mit wenig Ether versetzt, worauf 52 g (20 %) des dimeren p-Trichlormethylbenzoylcyanids auskristallisieren und abgesaugt werden. Aus dem Filtrat lassen sich durch Vakuumdestillation 127 g (48 % d. Th.) p-Trichlormethylbenzoylcyanid gewinnen.

| | | | | | | |
|---|---|---|---|---|---|---|
| $C_9H_4Cl_3NO$ | ber. : | C 43,50 | H 1,62 | N 5,64 | O 6,44 | Cl 42,80 |
| [248,50] | gef. : | C 43,6 | H 1,7 | N 5,5 | O 6,3 | Cl 42,4 |

Sdp. : 110 °C/0,1 Torr

$^1$H-NMR-Spektrum ($CDCl_3$) : δ = 8,2 (s)

IR-Spektrum ($CH_2Cl_2$) : $\tilde{\nu}$ = 1 680, 2 220 $cm^{-1}$

| | | | | | | |
|---|---|---|---|---|---|---|
| $C_{18}H_8Cl_6N_2O_2$ | ber. : | C 43,50 | H 1,62 | N 5,64 | O 6,44 | Cl 42,80 |
| [496,99] | gef. : | C 44,0 | H 1,9 | N 5,6 | O 6,6 | Cl 41,9 |

Schmp. : 182-183 °C

$^1$H-NMR-Spektrum ($CDCl_3$) : δ = 8,0 (AB, J = 9 Hz, 4 H), 8,05 (S,4 H)

IR-Spektrum ($CH_2Cl_2$) : $\tilde{\nu}$ = 1 750, 2 240 $cm^{-1}$

## Beispiel 2

Herstellung des 3,5-Bis(trichlormethyl)benzoylcyanids

78 g des 3,5-Bis(trichlormethyl)benzoylchlorids und 0,4 g Dimethylbenzylamin werden in 500 ml Methylenchlorid gelöst und bei – 15 °C zuerst eine Lösung von 10 ml Cyanwasserstoff in 50 ml

Methylenchlorid, anschließend eine Lösung von 12,4 g Kaliumcyanid in 20 ml Wasser zugetropft. Nach 1 1/2 Stunden wird durch Einrühren von Natriumsulfat getrocknet, von den anorganischen Salzen abgesaugt und das Lösungsmittel im Vakuum abgezogen. Aus dem zurückbleibenden zähen Rückstand kristallisieren auf Etherzusatz 14 g (18 % d. Th.) dimeres 3,5-Bis(trichlormethyl)-benzoylcyanid. Das verbleibende monomere Produkt wird durch doppelte Kugelrohrdestillation gereinigt.

$C_{10}H_3Cl_6NO$ ber. : C 32,83 H 0,83 N 3,83 Cl 58,14
[365,86] gef. : C 32,0 H 1,2 N 1,6 Cl 58,3
Sdp. : 155 °C/0,1 Torr
$^1$H-NMR-Spektrum ($CCl_4$) : $\delta$ = 8,5-8,9 (m)
IR-Spektrum ($CH_2Cl_2$) : $\tilde{\nu}$ = 1 680, 2 220 $cm^{-1}$
$C_{20}H_6Cl_{12}N_2O_2$ ber. : C 32,83 H 0,83 N 3,83 Cl 58,14
[731,72] gef. : C 32,6 H 1,0 N 3,4 Cl 57,7
Schmp. 180 °C
$^1$H-NMR-Spektrum ($CDCl_3$) : $\delta$ = 8,5 (d, J = 2 Hz, 2 H)
8,65-8,9 (m, 4 H)
IR-Spektrum ($CH_2Cl_2$) : $\tilde{\nu}$ = 1 760, 2 220 $cm^{-1}$

Tabelle I

Beispiele für Verbindungen der allgemeinen Formel (IV)

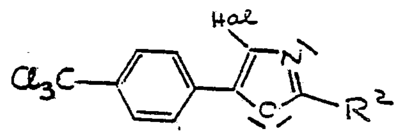

(IV)

(d. h. Formel I mit $R^1$ = H, und n = 1)

| Verbindung Nr. | $R^2$ = | Hal = | Schmp. (° C) | Langwelliges Absorptions-maximum in DMF $\lambda_{max}$(lg ε) [nm] |
|---|---|---|---|---|
| 1 | 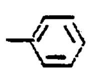 | Cl | 141-43 | 328 (4,49) |
| 2 | 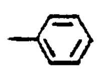 | Br | 134-35 | 328 (4,47) |
| 3 | 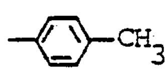 | Cl | 153-54 | 332 (4,51) |
| 4 | 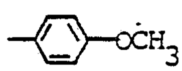 | Cl | 141-43 | 336 (4,53) |
| 5 | 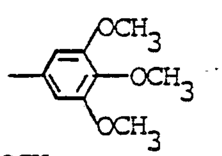 | Cl | 190-91 | 340 (4,52) |
| 6 | 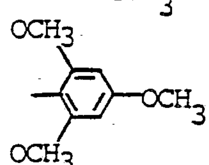 | Cl | 190-91 | 338 (4,53) |
| 7 | 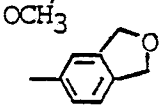 | Cl | 164-66 | 343 (4,51) |
| 8 | 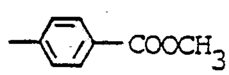 | Cl | 190-91 | 340 (4,52) |

(Fortsetzung)

| Verbindung Nr. | $R^2$ = | Hal = | Schmp. (° C) | Langwelliges Absorptions-maximum in DMF $\lambda_{max}$(lg ε) [nm] |
|---|---|---|---|---|
| 9 | 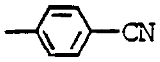 | Cl | 201 | 341 (4,51) |
| 10 | 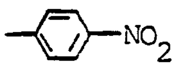 | Cl | 222-25 | 356 (4,37) |
| 11 | 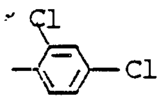 | Cl | 148-49 | 322 (4,45)<br>334 (S, 4,42) |
| 12 | 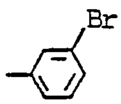 | Cl | 133-35 | 322 (4,49)<br>331 (4,49) |
| 13 | 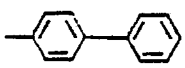 | Cl | 186-88 | 341 (4,62) |
| 14 | 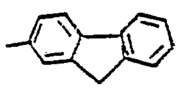 | Cl | 225 | 353 (4,68) |
| 15 | 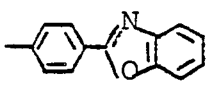 | Cl | 261-63 | 360 (4,73) |
| 16 | 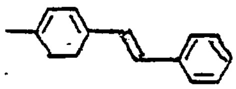 | Cl | 189-90 | 366 (4,76) |
| 17 | 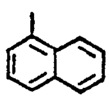 | Cl | 167-69 | 343 (4,41) |
| 18 | 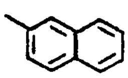 | Cl | 180-81 | 344 (4,54) |
| 19 | 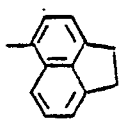 | Cl | 174-76 | 367 (4,44) |
| 20 | 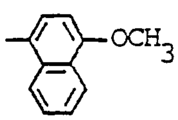 | Cl | 205-07 | 357 (4,45) |
| 21 | 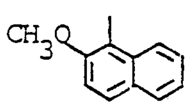 | Cl | 144-46 | 305 (4,31)<br>334 (S, 4,09) |
| 22 | 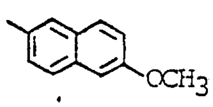 | Cl | 160-62 | 352 (4,57) |

(Fortsetzung)

| Verbindung Nr. | $R^2$ = | Hal = | Schmp. (° C) | Langwelliges Absorptions-maximum in DMF $\lambda_{max}$(lg ε) [nm] |
|---|---|---|---|---|
| 23 | 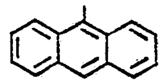 | Cl | 190-91 | 387 (4,05) |
| 24 | 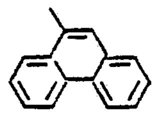 | Cl | 186-87 | 344 (4,42) |
| 25 | 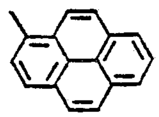 | Cl | 220-22 | 387 (4,61)<br>398 (4,59) |
| 26 | 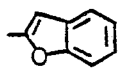 | Cl | 174-76 | 350 (4,61) |
| 27 | 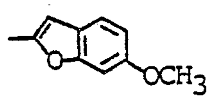 | Cl | 154-56 | 361 (4,61) |
| 28 | 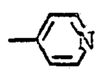 | Cl | 105-06 | 329 (4,49) |
| 29 | 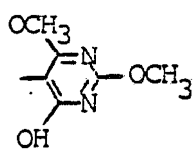 | Cl | 222-24 | 290 (4,28) |
| 30 | 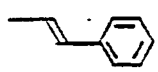 | Cl | 165 | 350 (4,56) |
| 31 | 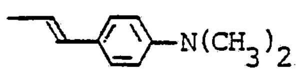 | Cl | ~ 190 | 406 (4,56) |
| 32 | 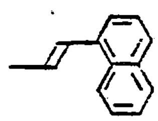 | Cl | 165-67 | 370 (4,50) |
| 33 | 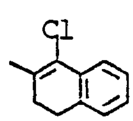 | Cl | 134-35 | 367 (4,52) |
| 34 | 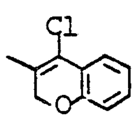 | Cl | 171-73 | 380 (4,45) |
| 35 | 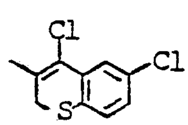 | Cl | 164-65 | 392 (4,18) |

Tabelle II

Beispiele für die Verbindungen der allgemeinen Formel (V)

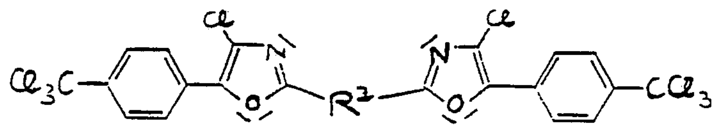 (V)

(d. h. Formel I mit $R^1$ = H, Hal = Cl, n = 2)

| Verbindung Nr. | $R^2$ = | Schmp. (° C) | Langwelliges Absorptions-maximum in DMF $\lambda_{max}(\lg \varepsilon)$ [nm] |
|---|---|---|---|
| 36 | 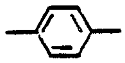 | 267-71 | 373 (4,73) |
| 37 | 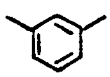 | 244-45 | 334 (4,78) |
| 38 | 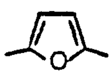 | 238-40 | 386 (4,68) |
| 39 | 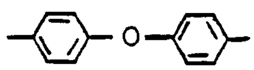 | 194-95 | 338 (4,84) |

Tabelle III

Beispiele für die Verbindungen der allgemeinen Formel (VI)

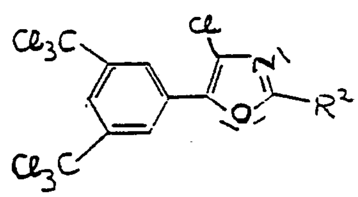 (VI)

(d. h. Formel I mit $R^1$ = $CCl_3$, Hal = Cl, n = 1)

| Verbindung Nr. | $R^2$ = | Schmp. (° C) | Langwelliges Absorptions-maximum in DMF $\lambda_{max}(\lg \varepsilon)$ [nm] |
|---|---|---|---|
| 40 | 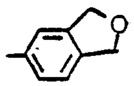 | 163 | 341 (4,43) |
| 41 | 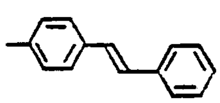 | 186-87 | 363 (4,74) |
| 42 | 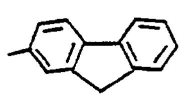 | 254-55 | 351 (4,64) |

Beispiel 3 (Verbindung 1)

Eine Lösung von 10 g Trichlormethylbenzoylcyanid (10 % Überschuß) und 3,8 g Benzaldehyd in 40 ml trockenem Tetrahydrofuran wird bei – 20 °C bis – 30 °C mit Chlorwasserstoffgas gesättigt. Nach 15 Std. bei 0 °C wird auf Eis gegossen und das ausgefallene Produkt aus Aceton umkristallisiert.

Ausbeute 10 g (74 % d. Th.)

| | | | | | |
|---|---|---|---|---|---|
| $C_{16}H_9Cl_4NO$ | ber. : | C 51,51 | H 2,43 | N 3,75 | Cl 38,01 |
| [373,07] | gef. : | C 51,4 | H 2,6 | N 3,7 | Cl 38,2 |

Schmp. : 141-143 °C

$^1$H-NMR-Spektrum : δ = 7,5 (m, 3 H), 8,0 (s, 4 H), 8,1 (m, 2 H)

UV-Spektrum (DMF) : $\lambda_{max}$ (ε) = 320 (30500), 328 (30800), 344 nm (S, 17200)

Beispiel 4 (Verbindung 2)

Eine Lösung von 10 g p-Trichlormethylbenzoylcyanid und 3,8 g Benzaldehyd in 40 ml Diethylether wird bei – 30 °C mit Bromwasserstoffgas gesättigt. Nach 15 Std. bei 0 °C wird auf Eis gegossen und das angefallene Produkt aus Aceton umkristallisiert.

Ausbeute 8,1 g (54 % d. Th.)

| | | | | | |
|---|---|---|---|---|---|
| $C_{16}H_9BrCl_3NO$ | ber. : | C 46,03 | H 2,17 | N 3,35 | Cl 25,47 |
| [417,52] | gef. : | C 45,8 | H 2,2 | N 3,1 | Cl 25,1 |

Schmp. : 134-135 °C

$^1$H-NMR-Spektrum ($CDCl_3$) : δ = 7,5 (m, 3 H), 8,0 (s, 4 H), 8,1 (m, 2 H)

UV-Spektrum (DMF) : $\lambda_{max}$ (ε) = 321 (29500), 328 nm (29500)

Beispiel 5 (Verbindung 4)

10 g p-Trichlormethylbenzoylcyanid und 8,1 g Anisaldehyd werden nach Beispiel 3 umgesetzt.

Ausbeute 9,4 g (58 % d. Th.)

| | | | | | |
|---|---|---|---|---|---|
| $C_{17}H_{11}Cl_4NO_2$ | ber. : | C 50,66 | H 2,75 | N 3,47 | Cl 35,18 |
| [403,09] | gef. : | C 50,9 | H 2,8 | N 3,4 | Cl 34,9 |

Schmp. : 141-143 °C

$^1$H-NMR-Spektrum ($CDCl_3$) : δ = 3,85 (s, $OCH_3$), 6,95 (d, J = 9 Hz, 2 H), 8,0 (d, J = 9 Hz, 2 H), 8,0 (s, 4 H)

UV-Spektrum (DMF) : $\lambda_{max}$ (ε) = 336 nm (33700)

Beispiel 6 (Verbindung 9)

4,7 g p-Cyanobenzaldehyd wird nach Beispiel 3, jedoch in Diethylether umgesetzt.

Ausbeute 10,5 g (73 % d. Th.)

| | | | | | |
|---|---|---|---|---|---|
| $C_{17}H_8Cl_4N_2O$ | ber. : | C 51,29 | H 2,03 | N 7,04 | Cl 35,62 |
| [398,08] | gef. : | C 51,4 | H 2,2 | N 7,1 | Cl 36,2 |

Schmp. : 201 °C

$^1$H-NMR-Spektrum ($CDCl_3$) : δ = 7,75 (d, J = 8 Hz, 2 H), 8,0 (s, 4 H), 8,2 (d, J = 8 Hz, 2 H)

UV-Spektrum (DMF) : $\lambda_{max}$ (ε) = 333 (S, 31400), 341 nm (32500)

IR-Spektrum ($CH_2Cl_2$) : $\tilde{\nu}$ = 2 220 $cm^{-1}$

Beispiel 7 (Verbindung 17)

10 g p-Trichlormethylbenzoylcyanid und 6,9 g (10 % Überschuß) 1-Naphthaldehyd werden bei – 15 °C in Tetrahydrofuran mit Chlorwasserstoffgas gesättigt. Nach 15 Std. bei 0 °C wird auf Eis gegossen und das ausgefallene Produkt aus Aceton umkristallisiert.

Ausbeute 12,7 g (75 % d. Th.)

| | | | | | |
|---|---|---|---|---|---|
| $C_{20}H_{11}Cl_4NO$ | ber. : | C 56,77 | H 2,62 | N 3,31 | Cl 33,52 |
| [423,13] | gef. : | C 57,1 | H 2,8 | N 3,5 | Cl 33,5 |

Schmp. : 167-169 °C

$^1$N-NMR-Spektrum ($CDCl_3$) : δ 7,55 (m, H), 7,6 (m, 2 H), 7,95 (m, 2 H), 8,05 (s, 4 H), 8,3 (dd, J = 1,5 Hz, J = 7 Hz, H), 9,25 (dm, J = 8 Hz, H)

UV-Spektrum (DMF) : $\lambda_{max}$ (ε) = 293 (13000), 343 nm (25600)

Beispiel 8 (Verbindung 26)

6,4 g Benzofuran-2-aldehyd werden nach beispiel 7 umgesetzt.

Ausbeute 8,8 g (53 % d. Th.)

$C_{18}H_9Cl_4NO_2$ ber. : C 52,34 H 2,20 N 3,39 Cl 34,33

[413,09] gef. : C 52,5 H 2,2 N 3,5 Cl 34,2

Schmp. : 174-176 °C

$^1$H-NMR-Spektrum ($CDCl_3$) : δ = 7,5 (m, 5 H), 8,0 (s, 4 H)

UV-Spektrum (DMF) : $\lambda_{max}$ (ε) = 350 (40500), 370 nm (S, 24000)

Beispiel 9 (Verbindung 31)

10 g p-Trichlormethylbenzoylcyanid (25 % Überschuß) und 5,3 g 4-Dimethylaminozimtaldehyd werden in 40 ml Diethylether bei – 30 °C mit Chlorwasserstoffgas gesättigt. Nach 48 Std. bei 0 °C wird auf Eis gegossen. Es wird in Methylenchlorid aufgenommen, mit Sodalösung behandelt, mit Wasser nachgewaschen, über Natriumsulfat getrocknet, eingeengt und aus Essigester umkristallisiert.

Ausbeute 7,8 g (58 % d. Th.)

$C_{20}H_{16}Cl_4N_2O$ ber. : C 54,33 H 3,65 N 6,34 Cl 32,07

[442,17] gef. : C 54,4 H 3,7 N 6,2 Cl 31,6

Schmp. : > 190 °C (Zers.)

$^1$H-NMR-Spektrum ($CDCl_3$) : δ = 3,0 [s, $N(CH_3)_2$], 6,66 (d, J = 16 Hz, H), 6,69 (d, J = 9 Hz, 2 arom. H), 7,44 (d, J = 9 Hz, 2 arom. H), 7,56 (d, J = 16 Hz, H), 7,97 (s, 4 arom. H)

UV-Spektrum (DMF) : $\lambda_{max}$ (ε) = 310 (16500), 406 nm (36100)

Beispiel 10 (Verbindung 36)

10 g p-Trichlormethylbenzoylcyanid und 2,7 g Terephthaldialdehyd werden bei – 10 °C in Tetrahydrofuran mit Chlorwasserstoffgas umgesetzt. Nach 15 Std. bei 0 °C wird auf Eis gegossen, abgesaugt und das schwer lösliche Produkt mit Dimethylformamid ausgekocht.

Ausbeute 5 g (38 % d. Th.)

$C_{26}H_{12}Cl_8N_2O_2$ ber. : C 46,75 H 1,81 N 4,19 Cl 42,46

[668,02] gef. : C 47,0 H 2,0 N 4,4 Cl 41,9

Schmp. : 267-271 °C

$^1$H-NMR-Spektrum (DMSO-$d_6$, 140 °C) : δ = 8,07 (s, 4 H), 8,23 (s, 8 H)

UV-Spektrum (DMF) : $\lambda_{max}$ (ε) = 317 (25800), 358 (S, 49000),
373 (54000), 390 nm (S, 34400)

Beispiel 11 (Verbindung 39)

15 g p-Trichlormethylbenzoylchlorid und 4,6 g 4,4'-Diformyldiphenylether werden in 40 ml Tetrahydrofuran bei – 30 °C bis – 20 °C in Anwesenheit von Chlorwasserstoffgas umgesetzt. Hydrolyse mit Eis und Umkristallisieren aus Toluol/Hexan ergeben 8,4 g (55 % d. Th.).

$C_{32}H_{16}Cl_8N_2O_3$ ber. : C 50,56 H 2,12 N 3,69 Cl 37,31

[760,12] gef. : C 50,5 H 2,4 N 3,4 Cl 36,4

Schmp. : 194-195 °C

$^1$H-NMR-Spektrum ($CDCl_3$) : δ = 7,1 (d, J = 9 Hz, 4 H), 7,95 (s, 8 H), 8,05 (d, J = 9 Hz, 4 H)

UV-Spektrum (DMF) : $\lambda_{max}$ (ε) = 338 nm (68900)

Beispiel 12 (Verbindung 40)

8 g des 3,5-Bistrichlormethylbenzoylcyanids und 2,4 g Piperonal werden bei – 30 °C bis – 20 °C in 40 ml trockenem Tetrahydrofuran mit Chlorwasserstoffgas umgesetzt. Nach 15 Std. bei 0 °C wird auf Eis gegossen, abgesaugt und aus Aceton umkristallisiert.

Ausbeute 2,1 g (25 % d. Th.)

$C_{18}H_8Cl_7NO_3$ ber. : C 40,45 H 1,51 N 2,62 Cl 46,44

[534,44] gef. : C 40,6 H 1,7 N 2,3 Cl 45,4

Schmp. : 163 °C

$^1$H-NMR-Spektrum ($CDCl_3$) : δ = 6,06 (s, $CH_2$), 6,92 (d, J = 8 Hz, H), 7,52 (d, J = 2 Hz, H), 7,66 (dd, J = 2 Hz, J = 8 Hz, H), 8,47 (t, J = 2 Hz, H), 8,53 (d, J = 2 Hz, 2 H)

UV-Spektrum (DMF) : $\lambda_{max}$ (ε) = 341 nm (26700)

Beispiel 13

Eine elektrochemisch aufgerauhte Aluminiumplatte wird mit einer Lösung bestehend aus :

6,5 Gt Trimethylolethantriacrylat

6,5 Gt Methacrylsäure/Methylmethacrylat-Copolymerem (Säurezahl 115)

0,2 Gt Photoinitiator 1

64,0 Gt Ethylenglykolmonoethylether
22,6 Gt Butylacetat
0,2 Gt 2,4-Dinitro-6-chlor-2'-acetamido-5'-methoxy-4'-(β-hydroxyethyl-β'-cyanoethyl)amino-azobenzol

beschichtet, so daß sich ein Schichtgewicht von 3 bis 4 mg/m$^2$ nach dem Trocknen ergibt. Diese beschichtete Platte wird zusätzlich mit einem Polyvinylalkoholdeckstrich von 4 μm Dicke versehen und in einem Vakuumbelichtungsrahmen mit Leuchtröhren TLAK 20W/05 der Firma Philips aus einem Abstand von ca. 10 cm kontaktbelichtet. Das negative Bild der Vorlage wird mit einer 1,5 %igen wäßrigen Natriummetasilikatlösung entwickelt.

In Tabelle IV ist die Anzahl der ausgehärteten Stufen eines Stufenkeils aufgeführt, wobei der Photoinitiator 1 durch eine äquimolare Menge eines anderen Photoinitiators ersetzt wird. Eine Differenz von zwei Keilstufen entspricht dabei der doppelten Lichtempfindlichkeit der lichtempfindlichen Schicht.

Tabelle IV

| Verbindung Nr. | Ausgehärtete Keilstufen nach Beispiel 13 (t = Belichtungszeit) | |
|---|---|---|
| | t = 45 sec | t = 2 min |
| 1 | 5 | 9 |
| 2 | 2 | 6 |
| 3 | 6 | 9 |
| 4 | 8 | 11 |
| 5 | 8 | 11 |
| 6 | 7 | 10 |
| 7 | 7 | 10 |
| 8 | 8 | 10 |
| 9 | 6 | 9 |
| 10 | 8 | 11 |
| 11 | 6 | 9 |
| 12 | 6 | 10 |
| 13 | 9 | 12 |
| 14 | 9 | 11 |
| 16 | 7 | 11 |
| 17 | 9 | 11 |
| 18 | 9 | 11 |
| 19 | 8 | 10 |
| 20 | 9 | 11 |
| 21 | 6 | 9 |
| 22 | 10 | 12 |
| 23 | 0 | 1 |
| 24 | 8 | 11 |
| 25 | 4 | 8 |
| 26 | 8 | 11 |
| 27 | 11 | 13 |
| 28 | 5 | 7 |
| 29 | 7 | 10 |
| 30 | 7 | 10 |
| 31 | 3 | 6 |
| 32 | 3 | 5 |
| 33 | 7 | 11 |
| 34 | 1 | 4 |
| 37 | 5 | 8 |
| 38 | 4 | 8 |
| 39 | 8 | 10 |
| 40 | 5 | 7 |
| 41 | 1 | 4 |
| 42 | 1 | 4 |
| Bis(trichlormethyl)-(4-methoxystyryl)-s-triazin nach DE-A-22 43 621 | 5 | 8 |
| 9-Phenylacridin nach DE-C-20 27 467 | 10 | 13 |

Die in der Praxis eingesetzte, ebenfalls eine Trihalogenmethylgruppe aufweisende Verbindung gemäß der DE-OS-2 243 621 wird von einer großen Anzahl an erfindungsgemäßen Produkten in der Wirksamkeit übertroffen. Die ebenfalls in der Praxis eingesetzte, keine Trihalogenmethylgruppe aufweisende Verbindung gemäß der DE-C-2 027 467 ist in etwa mit der Wirksamkeit der erfindungsgemäßen Verbindungen 25 und 30 vergleichbar, sie kann jedoch nur in strahlungsempfindlichen Massen eingesetzt werden, die photopolymerisierbare Monomere enthält, da das photolytisch-abspaltbare Halogen fehlt, d. h. ein Einsatz in strahlungsempfindlichen Massen, die eine Verbindung enthalten, deren Löslichkeit durch Einwirkung von Säure verändert wird, kann — im Gegensatz zu den erfindungsgemäßen Verbindungen — dann nicht erfolgen.

Beispiel 14

Eine Schichtzusammensetzung gemäß Beispiel 13 unter Verwendung von 0,24 Gt der lichtempfindlichen Verbindung 13 wird vor der Belichtung einem Lagertest bei 100 °C unterworfen. Wie Tabelle V zeigt, läßt sich kein Aktivitätsverlust feststellen.

Tabelle V

| Lagerzeit [min] bei 100 °C vor der Belichtung | ausgehärtete Keilstufen unter Verwendung der Initiators 14 (t = 2 min) |
|---|---|
| 0 | 9 |
| 15 | 9 |
| 30 | 9 |
| 45 | 9 |
| 60 | 9 |
| 75 | 7 |
| 90 | 8 |
| 120 | 8 |
| 150 | 8 |
| 180 | 8 |
| 210 | 9 |
| 240 | 9 |

Beispiel 15

Eine elektrochemisch aufgerauhte Aluminiumplatte wird mit einer Lösung bestehend aus

6,5 Gt Trimethylolethantriacrylat
6,5 Gt Methacrylsäure/Methylmethacrylat-Copolymerem (Säurezahl 115)
0,24 Gt Photoinitiator 13
64,0 Gt Ethylenglykolmonomethylether
22,6 Gt Butylacetat
0,24 Gt Leukomalachitgrün

beschichtet und mit einem Polyvinylalkohol-Deckstrich versehen. Nach einer Belichtungszeit von 60 sec gemäß Beispiel 13 wird ein negatives, grünes Bild der Vorlage erhalten, das durch Entwicklung zur druckfähigen Platte fixiert wird. Wird Leukomalachitgrün durch Kresolrot ersetzt, so erhält man ein rotes Bild der Vorlage.

Beispiel 16

Ein gebürstetes Aluminiumblech wird im Tauchverfahren mit einer Lösung bestehend aus

10 Vt Methylethylketon
1 Gt Kresol-Formaldehyd-Novolak
0,3 Gt Triethylenglykol-Ethylbutyraldehyd-Polykondensat
0,015 Gt einer der erfindungsgemäßen Verbindungen

beschichtet, gemäß Beispiel 13 belichtet und das positive Bild der Volage mit einer

5,5 Gt Natriummetasilikat · 9 $H_2O$
3,4 Gt Trinatriumphosphat · 12 $H_2O$
0,4 Gt Natriumdihydrogenphosphat
90,7 Gt entsalztes Wasser

enthaltenden Lösung entwickelt.

In Tabelle VI ist die Anzahl der entwickelbaren Keilstufen angegeben.

Tabelle VI

| Verbindung Nr. | Keilstufen (t = 2 min) | Verbindung Nr. | Keilstufen (t = 2 min) |
|---|---|---|---|
| 1 | 5 | 25 | 9 |
| 2 | 4 | 26 | 0 |
| 3 | 5 | 27 | 6 |
| 4 | 5 | 28 | 5 |
| 5 | 8 | 29 | 8 |
| 6 | 7 | 30 | 9 |
| 7 | 11 | 31 | 0 |
| 8 | 3 | 32 | 8 |
| 9 | 3 | 33 | 6 |
| 10 | 3 | 34 | 1 |
| 11 | 6 | 35 | 8 |
| 12 | 3 | 36 | 9 |
| 13 | 5 | 37 | 7 |
| 14 | 9 | 38 | 1 |
| 15 | 5 | 40 | 4 |
| 16 | 10 | 41 | 3 |
| 17 | 7 | 44 | 3 |
| 18 | 7 | 45 | 7 |
| 19 | 8 | Bis(trichlormethyl)- | |
| 20 | 9 | (4-methoxystyryl)-s-triazin | |
| 21 | 7 | nach DE-A-22 43 621 | |

Die in der Praxis eingesetzte Verbindung gemäß der DE-A-2 243 621 wird von vielen der erfindungsgemäßen Produkte in der Wirksamkeit erreicht und zum Teil übertroffen ; in Photopolymerschichten ist die bekannte Verbindung jedoch weniger wirksam (s. Tabelle IV).

Beispiel 17

Eine elektrochemisch aufgerauhte Aluminiumplatte wird mit einer Lösung bestehend aus zugemischt. Nach der Belichtung wird ein rot-gefärbtes Bild der Vorlage erhalten.

Beispiel 18

Einer elektrochemisch aufgerauhte Aluminiumplatte wird mit einer Lösung bestehend aus

1,5 Gt Bisphenol-A-diglycidylether
1,5 Gt Kresol-Formaldehyd-Novolak
0,1 Gt Verbindung 14
0,01 Gt Kristallviolett
40,5 Gt Butanon-2

beschichtet und gemäß Beispiel 13 wird 5 min belichtet. Es entsteht ein negatives Bild der Vorlage, das mit einer wäßrigen Lösung, die

0,6 Gew.-% Natriumhydroxid
0,52 Gew.-% Natriummetasilikat
0,8 Gew.-% Butanol

enthält, zur druckfähigen Platte entwickelt wird.

Beispiel 19

Gemäß Beispiel 13 wird eine Schicht unter Verwendung des Photoinitiators 18 hergestellt und unterschiedlichen Belichtungszeiten ausgesetzt. Die Anzahl der jeweils ausgehärteten Keilstufen steigt linear mit der Belichtungsdauer (Tabelle VII).

Tabelle VII

| Belichtungsdauer (sec) | ausgehärtete Keilstufen |
|---|---|
| 10 | 2 |
| 20 | 4 |
| 40 | 6 |
| 80 | 8 |
| 160 | 10 |
| 320 | 12 |

**Patentansprüche**

1. 4-Halogen-5-(trichlormethyl-phenyl)-oxazol-Derivate der allgemeinen Formel (I)

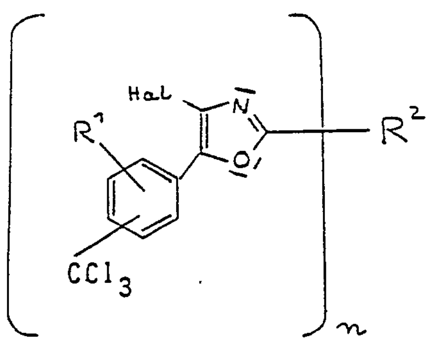

(I)

in der bedeuten

Hal ein Halogenatom,

n die Zahl 1 oder 2,

$R^1$ ein Wasserstoffatom oder eine weitere Gruppe $CCl_3$ und

$R^2$ einen n-wertigen, unsubstituierten oder durch Dialkylaminogruppen mit 2 bis 6 Kohlenstoffatomen, Halogenatome, Nitro-, Cyano-, Sulfonyl-, Carboxy-, Trifluormethyl-, Alkyl-, Cycloalkyl-, Alkoxy-, Alkoxycarbonyl-, Alkoxycarbonimidoyl-, Carboxyamido-, Phenyl-, Naphthyl-, Indenyl-, Fluorenyl-, Anthryl-, Phenanthryl, Pyrenyl, Biphenylyl-, Stilbenyl-, Styryl-, Furyl-, Benzofuryl-, Dibenzofuryl-, Pyrrolyl-, Indolyl-, Carbazolyl-, Thienyl-, Benzothienyl-, Imidazolyl-, Benzimidazolyl-, Oxazolyl-, Benzoxazolyl-, Isoxazolyl-, Thiazolyl, Benzthiazolyl-, Triazolyl-, Oxdiazolyl-, Thiadiazolyl-, Pyridyl-, Chinolyl-, Isochinolyl-, Acridyl-, Pyrimidyl-, Benzopyranyl-, Benzothianyl- oder Phenoxygruppen substituierten, maximal vierkernigen aromatischen oder heteroaromatischen, gegebenenfalls partiell hydrierten Rest, der an einem ungesättigten Ring-C-Atom direkt oder über eine bis zu vier ausschließlich olefinisch ungesättigten C-Atome enthaltenden Kette mit dem Oxazolyl-Teil des Moleküls gemäß der allgemeinen Formel (I) verbunden ist.

2. Verbindungen der allgemeinen Formel I nach Anspruch 1, in der

n die Zahl 1 oder 2 und

Hal ein Chlor- oder Bromatom

bedeuten.

3. Verbindungen der allgemeinen Formel

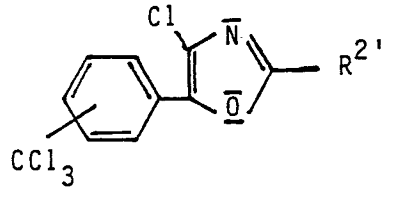

in der $R^{2'}$ einen gemäß Anspruch 1 substituierten oder unsubstituierten Phenyl-, Benzofuranyl- oder Naphthylrest bedeutet.

4. Verwendung einer Verbindung nach einem der Ansprüche 1 bis 3 als strahlungsempfindliche Verbindung in einer strahlungsempfindlichen Masse.

5. Strahlungsempfindliche Masse, die a) als strahlungsempfindliche Verbindung 0,1 bis 10 Gew.-% einer Verbindung nach einem der Ansprüche 1 bis 3 und b) eine ethylenisch ungesättigte Verbindung, die eine durch freie Radikale ausgelöste Polymerisation eingehen kann, eine Verbindung, deren Löslichkeit

durch Einwirkung von Säure verändert wird, oder eine Verbindung enthält, die durch saure Katalysatoren zur kationischen Polymerisation veranlaßt wird.

6. Strahlungsempfindliche Masse nach Anspruch 5, dadurch gekennzeichnet, daß sie als Verbindung, deren Löslichkeit durch Einwirkung von Säuren erhöht wird, eine Verbindung mit mindestens einer durch Säure spaltbaren C-O-C-Gruppierung enthält.

7. Verfahren zur Herstellung der Verbindungen nach Anspruch 1, dadurch gekennzeichnet, daß man ein Trichlormethyl-benzoylcyanid der allgemeinen Formel (II) mit einem Aldehyd der allgemeinen Formel (III)

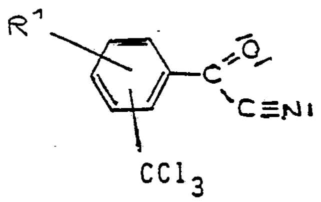 (II) 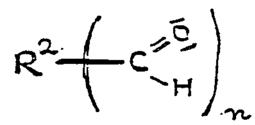 (III)

unter Einwirkung von HHal umsetzt und die Reaktionsprodukte durch Hydrolyse freisetzt, wobei die Bezeichnungen Hal, n, $R^1$ und $R^2$ die in Anspruch 1 angegebene Bedeutung haben.

**Claims**

1. 4-halogeno-5-(trichloromethyl-phenyl)-oxazole derivatives of the general formula I

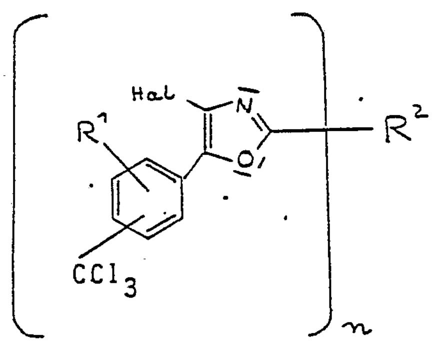 (I)

wherein

Hal represents a halogen atom,

n is 1 or 2,

$R^1$ represents a hydrogen atom or a further $CCl_3$ group, and

$R^2$ represents a n-valent, at most tetranuclear aromatic or heteroaromatic radical which may be partially hydrogenated and, at an unsaturated ring C-atom, is linked, directly or via a chain which contains up to 4 exclusively olefinically unsaturated C-atoms, to the oxazolyl part of the molecule according to the general formula I, which radical is unsubstituted or substituted by dialkylamino groups having 2 to 6 carbon atoms, halogen atoms, nitro, cyano, sulfonyl, carboxyl, trifluoromethyl, alkyl, cycloalkyl, alkoxy, alkoxycarbonyl, alkoxycarbonylimidoyl, carboxyamido, phenyl, naphthyl, indenyl, fluorenyl, anthryl, phenanthryl, pyrenyl, biphenylyl, stilbenyl, styryl, furyl, benzofuryl, dibenzofuryl, pyrrolyl, indolyl, carbazolyl, thienyl, benzothienyl, imidazolyl, benzimidazolyl, oxazolyl, benzoxazolyl, isoxazolyl, thiazolyl, benzothiazolyl, triazolyl, oxadiazolyl, thiadiazolyl, pyridyl, chinolyl, isochinolyl, acridyl, pyrimidyl, benzopyranyl, benzothianyl or phenoxy groups.

2. Compounds of the general formula I, as claimed in claim 1, wherein :

n is the number 1 or 2 and

Hal represents a chlorine or bromine atom.

3. Compounds of the general formula

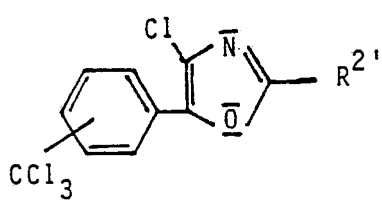

wherein $R^{2'}$ represents a phenyl, benzofuranyl or naphthyl group which is unsubstituted or substituted according to claim 1.

4. The use of a compound according to any one of claims 1 to 3, as a radiation-sensitive compound in a radiation-sensitive composition.

5. A radiation-sensitive composition which (a) as the radiation-sensitive compound, contains from 0.1 to 10 % by weight of a compound as claimed in any of claims 1 to 3 and (b) an ethylenically unsaturated compound which is capable of undergoing a polymerization reaction initiated by free radicals, a compound, the solubility of which is modified by the action of an acid or a compound which is caused by acid catalysts to undergo a cationic polymerization.

6. A radiation-sensitive composition as claimed in claim 5, which comprises as the compound the solubility of which is increased by the action of acids, a compound which has at least one C-O-C grouping, which can be split by acid.

7. A process for the preparation of the compounds as claimed in claim 1, which comprises reacting a trichloromethyl-benzoyl cyanide of the general formula (II) with an aldehyde of the general formula (III)

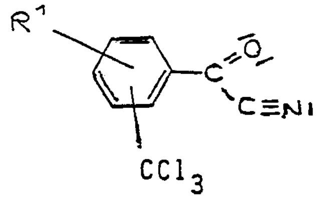 (II) 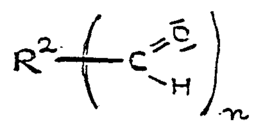 (III)

under the action of HHal and liberating the reaction product by hydrolysis, the symbols Hal, n, $R^1$ and $R^2$ having the meaning indicated in claim 1.

**Revendications**

1. Dérivés de 4-halogéno-5-(trichlorométhyl-phényl)-oxazole de formule générale (I)

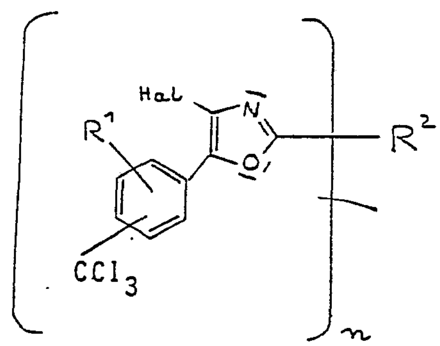 (I)

dans laquelle

Hal représente un atome d'halogène,

n est 1 ou 2,

$R^1$ représente un atome d'hydrogène ou un autre groupe $CCl_3$ et

$R^2$ représente un groupe aromatique ou hétéroatomique à 4 noyaux au maximum, éventuellement partiellement hydrogéné, de valence n, non substitué ou substitué par des groupes dialkylamino ayant de 2 à 6 atomes de carbone, des atomes d'halogène, des groupes nitro, cyano, sulfonyle, carboxy, trifluorométhyle, alcoyle, cycloalcoxy, alcoxy, alcoxycarbonyle, alcoxycarbonimidoyle, carboxyamido, phényle, naphtyle, indényle, fluorényle, anthryle, phénanthryle, pyrényle, biphénylyle, stilbényle, styryle, furyle, benzofuryle, dibenzofuryle, pyrrolyle, indolyle, carbozolyle, thiényle, benzothiényle, imidazolyle, benzimidazolyle, oxazolyle, benzoxazolyle, isoxazolyle, thiazolyle, benzothiazolyle, triazolyle, oxodiazolyle, thiadiazolyle, pyridyle, quinoléyle, isoquinoléyle, acridyle, pyrimidyle, benzopyrannyle, benzothianyle ou phénoxy, lequel groupe est relié sur un atome de carbone cyclique insaturé, directement ou par l'intermédiaire d'une chaîne contenant jusqu'à quatres atomes de carbone exclusivement à insaturation oléfinique, à la partie oxazolyle de la molécule de formule générale (I).

2. Composés de formule générale I selon la revendication 1, dans laquelle formule

n est 1 ou 2, et

Hal représente un atome de chlore ou de brome.

3. Composés de formule générale

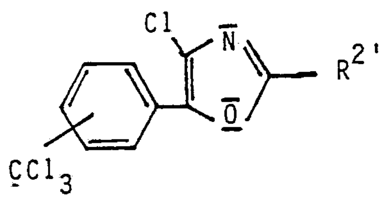

dans laquelle $R^{2'}$ représente un groupe phényle, benzofuranyle ou naphtyle substitué on non substitué selon la revendication 1.

4. Utilisation d'un composé selon l'une quelconque des revendications 1 à 3, en tant que composé sensible aux radiations, dans une composition sensible aux radiations.

5. Composition sensible aux radiations qui contient a) en tant que composé sensible aux radiations de 0,1 à 10 % en poids d'un composé selon l'une quelconque des revendications 1 à 3 et b) un composé à insaturation éthylénique qui peut induire une polymérisation par radicaux libres, un composé dont la solubilité est modifiée par action d'un acide, ou un composé qui donne lieu à une polymérisation cationique au moyen de catalyseurs acides.

6. Composition sensible aux radiations selon la revendication 5, caractérisée en ce que comme composé dont la solubilité est augmentée par action d'acides, elle contient un composé ayant au moins un groupe C-O-C scindable par un acide.

7. Procédé pour la préparation des composés selon la revendication 1, caractérisé en ce qu'on fait réagir un cyanure de trichlorométhyl-benzoyle de formule générale (II) avec un aldéhyde de formule générale (III)

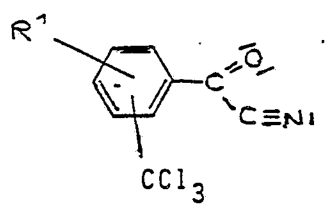 (II) 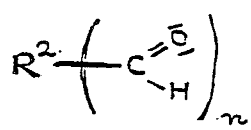 (III)

en présence de HHal et on libère les produits de réaction par hydrolyse, les symboles Hal, n, $R^1$ et $R^2$ ayant les significations indiquées dans la revendication 1.